Europäisches Patentamt

European Patent Office (11) Publication number: **0 075 838**

Office européen des brevets **A1**

(12) **EUROPEAN PATENT APPLICATION**

(21) Application number: 82108717.8

(22) Date of filing: 21.09.82

(51) Int. Cl.³: **C 07 C 43/04**
C 07 C 41/06, C 07 C 41/42

(30) Priority: 24.09.81 US 305292

(43) Date of publication of application:
06.04.83 Bulletin 83/14

(84) Designated Contracting States:
AT BE CH DE FR GB IT LI LU NL SE

(71) Applicant: PHILLIPS PETROLEUM COMPANY
5th and Keeler
Bartlesville Oklahoma 74004(US)

(72) Inventor: McCarthy, William Chase
2911 Sheridan Road
Bartlesville Oklahoma 74004(US)

(72) Inventor: Hutson, Thomas, Jr.
5300 Brock Drive
Bartlesville Ohlahoma 74003(US)

(74) Representative: Dost, Wolfgang, Dr.rer.nat.,
Dipl.-Chem. et al,
Patent- und Rechtsanwälte
Bardehle-Pagenberg-Dost-Altenburg & Partner Postfach
86 06 20
D-8000 München 86(DE)

(54) Process for the preparation of methyl tert-butyl ether.

(57) A novel process for the preparation of methyl tertiary butyl ether comprising reacting isobutene and methanol in an etherification zone and fractionating the product wherein part of the overhead is recycled to the etherification zone.

FIG. I

Croydon Printing Company Ltd.

1

## PROCESS FOR THE PREPARATION OF METHYL TERT-BUTYL ETHER

### Field of the Invention

The present invention relates to the production of methyl-tert-butyl ether from methanol and isobutylene.

### Background of Invention

In view of the growing need to limit the use of lead-containing additives as octane improvers for gasoline, there is an increasing need for more environmentally safe octane-improving additives. One of the more promising additives is methyl tert-butyl ether (MTBE).

It has long been known that MTBE can be produced from the reaction of isobutylene and methanol. See Leum et al, U. S. patent 2,480,940. In a commercial scale MTBE plant, it is obviously desirable to maximize the production of substantially pure MTBE without the need for numerous or expensive separation steps. It has previously been suggested that the MTBE yield could be increased by using excess amounts of either the isobutene or the methanol. Such techniques have, however, led to separation problems or undesirable by-products which adversely affect catalyst life.

An object of the present invention is to provide a method for producing MTBE in a system wherein the MTBE therein is readily separated into a substantially pure MTBE product.

Still another object of the present invention is to provide a process in which the catalyst activity can be maintained over long periods of time.

Other aspects, objects and advantages of the present invention will be apparent from the following disclosure and the attached Figures.

## Summary of the Invention

The present invention is an improvement in the process wherein a feedstream containing isobutylene and at least one hydrocarbon diluent capable of forming an azeotrope with methanol is combined with methanol and subjected to an etherification reaction wherein the isobutylene and the methanol react to produce an effluent containing methyl tertiary butyl ether, unreacted methanol, and diluent hydrocarbon. The effluent is then subjected to fractionation to produce a bottoms product comprising MTBE and an overhead product comprising methanol and unreacted hydrocarbons. The improvement of the present invention involves recycling to the etherification portions of the overhead that have not had the methanol separated therefrom. In an expecially preferred embodiment, the overhead is recycled in an amount sufficient to insure that substantially all the methanol in the etherization effluent will go overhead in the fractionation step.

## Brief Description of the Drawing

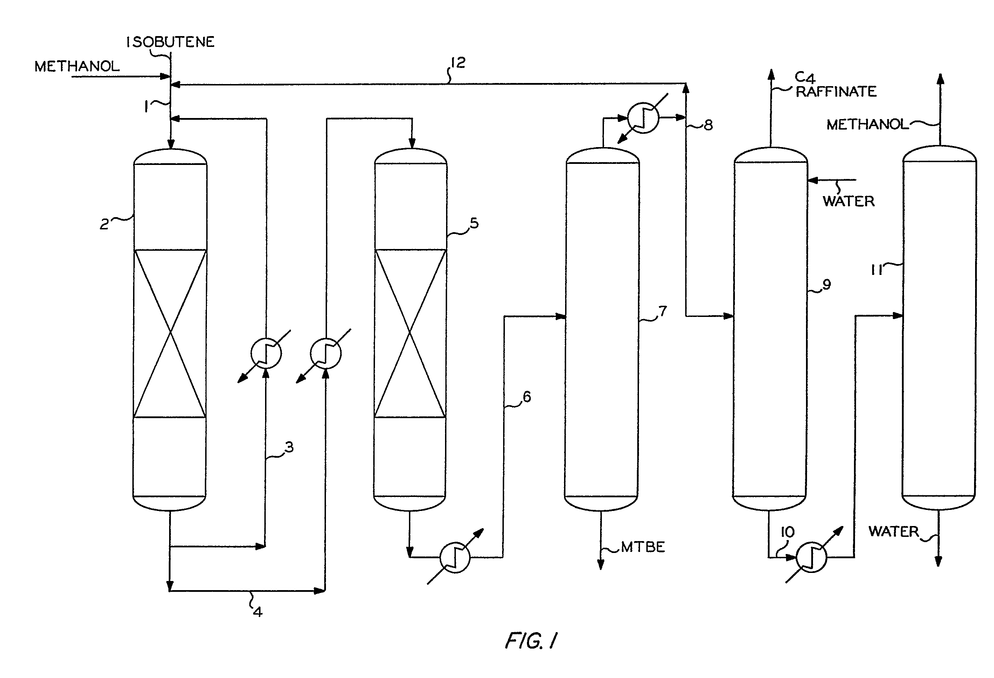

FIGURE 1 is a schematic illustration of a process employing the present invention.

## Detailed Description of the Invention

The conversion of the isobutylene, also referred to herein as isobutene, to methyl tert-butyl ether by reaction with methanol can be carried out in any suitable manner known in the art.

The etherification is preferably carried out in the presence of a solid resin etherification catalyst. Such catalysts include high molecular weight carbonaceous materials containing sulfonate groups. Typical of these catalysts are the sulfonated coals ("Zeo-Karb H", "Nalcite X" and "Nalcite AX") produced by the treatment of bituminous coals with sulfuric acid. These materials are usually available in a neutralized form and in this case must be activated to the hydrogen form by treatment with a strong mineral acid such as hydrochloric acid, followed by water-washing to remove sodium and chloride ions prior to use.

The sulfonated resin type catalysts are preferred for use in the present invention. These catalysts include the reaction products of phenol-formaldehyde resins and sulfuric acid ("Amberlite IR-1", "Amberlite IR-100", and "Nalcite MX"). Also useful are the sulfonated resinous polymers of coumarone-indene with furfural; sulfonated polymers of coumarone-indene with cyclopentadiene and furfural; and sulfonated polymers of cyclopentadiene with furfural.

The most preferred cationic exchange resins are strongly acidic exchange resins consisting essentially of sulfonated polystyrene resin: for instance, a divinylbenzene cross-linked polystyrene matrix having 0.5-20 percent and preferably 4-16 percent of copolymerized divinylbenzene therein, bearing ionizable or functional nuclear sulfonic acid groups. These resins are manufactured and sold commercially under various trade names such as "Dowex 50", "Nalcite HCR" and "Amberlyst 15". As commercially obtained they generally have a water content of about 50 percent and can be used as is or the water can be removed first. The resin particle size may typically be 10 to 50 mesh (United States Sieve Series).

Preferably, the etherification is conducted over a fixed bed of a particulate solid ion exchange resin such as Amberlyst 15. In an especially preferred embodiment, the etherification is conducted in the manner described in U. S. Patent Application No. 287,458, filed July 27, 1981 by Donald J. Mackovec, the disclosure of which is incorporated herein by reference.

The hydrocarbon-containing feedstream can be from any suitable source. For example, the $C_4$ fraction obtained from thermal cracking, steam cracking, or catalytic cracking can be employed. Examples of hydrocarbons capable of forming azeotropes with methanol include isobutane, n-butane and linear butenes. Such hydrocarbons are generally present in the $C_4$ fraction of cracking operations. Preferably, the hydrocarbon-containing feedstream contains enough of the azeotroping hydrocarbons that the fresh feedstream and the overhead stream from the fractionator can be combined to produce a feed for the first reactor having at least about 60 weight percent of said azeotroping hydrocarbons on a methanol free basis.

The present invention is particularly useful when the fresh feedstream consists essentially of $C_4$ hydrocarbons and the isobutene is present in said feedstream in an amount greater than about 25 weight percent. The invention is even more useful when the isobutene is 50 weight percent or more of the fresh feed.

It is desirable to have the methanol and the isobutylene present in a molar ratio of no more than about 1/1 in the etherification zone. Since occasionally some of the isobutene in the fresh feed may during the etherification dimerize or react with small amounts of water to form tert-butyl alcohol, the molar ratio of methanol to isobutene in the fractionator overhead may exceed 1/1. It is thus preferred to employ the fresh methanol in such an amount that the molar ratio of said methanol to the isobutene in the fresh feed is slightly less than 1/1. The optimum methanol ratio can obviously be met by monitoring the ratio downstream of all the combined streams and varying the flow of the fresh methanol as necessary.

If methanol is employed in amounts such that the molar ratio of methanol to isobutene in the etherification zone is greater than 1/1, it becomes necessary to have larger amounts of the other hydrocarbons present in order to obtain a effluent from which substantially all of the methanol can be taken overhead in a subsequent fractionation step.

A further understanding of the present invention will be provided by referring to the drawing which illustrates a preferred embodiment of the present invention.

In the drawing a $C_4$ olefinic stream containing isobutene is combined with methanol and passed through line 1 to a first reactor 2 containing a fixed bed of an acid ion exchange resin, such as Amberlyst 15. A portion of the effluent from reactor 2 is cooled and recycled via line 3 to the inlet of reactor 2. The cooling of recycled effluent is used to control the temperature of the combined streams at the inlet of reactor 2.

The yield portion of the effluent from reactor 1 is cooled and passed via line 4 to a reactor 5 also containing a fixed bed of acid ion exchange resin. The effluent from reactor 5 is heated and passed via line 6 to a fractionation column 7 wherein MTBE is separated as bottom product from methanol and unreacted portions of the $C_4$ feed.

The overhead from the fractionator, comprising methanol and unreacted portions of the feed, is cooled and a portion thereof is sent through line 12 to line 1 where it is combined with the fresh feedstream. The yield portion of the overhead is passed to a water wash column 9 wherein methanol is washed out of the unreacted portions of the $C_4$ feed.

The bottoms from wash column 9 comprising methanol and water is then heated and passed via line 10 to a fractionation column 11 wherein water and methanol are separated. Generally, the fractionation in column 11 can be carried out so as to result in methanol suitable for reuse as the methanol reactant stream that is passed into reactor 2.

In a typical operation, a $C_4$-containing feedstream containing more than about 25 weight percent isobutene, say for example about 50 weight percent is combined with methanol and passed to reactor 2. Reactor effluent and a portion of the overhead from fractionator 7 are also combined with the stream and passed into reactor 2. The methanol and isobutene content of the mixture entering reactor 2 is monitored and the fresh methanol flow adjusted as necessary to assure that the molar ratio of methanol to isobutene is about 1/1. The pressure in reactor 2 would typically be in the range of about 180 to about 235 psia. The effluent from reactor 2 is recycled via line 3 at such a rate that the temperature of the effluent at the outlet of reactor 2 would be in the range of about 155 to 160°F. Typically, the LHSV of the combined stream resulting from the fresh feed and the fractionator overhead recycle would be in the range of about 3 to about 7. Preferably, the first reaction zone is operated under conditions such that there is at least about 80 percent conversion of the isobutene.

The yield portion of the effluent from reactor 2 would be passed via line 4 to the second reactor 5. This stream would be cooled to a temperature in the range of about 100 to about 110°F prior to being passed to reactor 5. The effluent from reactor 5 at the outlet would preferably be only about 5 to 10°F greater than that of the inlet temperature. The pressure in reactor 5 would typically be about 170 to 210 psia. Preferably, the second reactor is operated under conditions such that there is at least 50 percent conversion of the remaining isobutene.

The effluent from reactor 5 is heated and passed to column 7. The column is typically operated at a pressure in the range of about 85 to about 125 psia. For example, at 85 psia, top temperature would be about 122°F, and bottom temperature about 236°F.

The yield portion of the overhead from the fractionator 7 is passed to the water wash or methanol extractor 9. Typical operating conditions for the extractor are a temperature of about 100°F and a pressure of about 120 psia.

The methanol-water mixture recovered from extractor 9 is passed to fractionator 11. Typically, fractionator 11 would be operated at pressures in the range of about 20 to about 50 psia. For example, at 25 psia, top temperature would be about 169°F, and bottom temperature would be about 240°F.

The recycling of overhead from fractionator 7 shifts the reaction equilibrium toward the MTBE product thus resulting in conversions in reactor 2 that are greater than would be obtained at those conditions if the overhead were not recycled. Since unreacted isobutene is recycled in the process, there is also provided a significant reduction in the amount of isobutene in the overhead from the wash column 9. Accordingly, a much more efficient conversion of isobutene is provided. Further, the recycling of the overhead from fractionator 7 provides additional hydrocarbons which will not react and will thus be present to help azeotrope the methanol overhead in fractionator 7. If a fresh feed of the type described, i.e., one having about 50 weight percent isobutylene, were passed through the reactors under conditions as above described, but without recycle of fractionator overhead, the amount of unreacted hydrocarbons in most cases would not be sufficient to allow substantially all of the methanol to be recovered overhead from fractionator 7. By controlling the amount of recycled fractionator overhead, it is thus possible to obtain MTBE that is substantially free of methanol even though a high weight percent isobutene feed is being employed.

For the purpose of further illustration assume that the fresh $C_4$ feedstream that is used in a process as illustrated in FIGURE 1 has the following composition:

| Isobutene | 50.0 wt. % |
|---|---|
| Isobutane | 45.0 |
| Light Ends | 2.0 |
| Heavy Ends | 2.97 |
| Water | 0.03 |

Based on the inventors' experience with processes of the type illustrated and their knowledge regarding how various hydrocarbons assist in bringing methanol overhead in fractionator 7, it is estimated that overall isobutene conversion to MTBE of 97.2 percent can be obtained as compared to 96.0 percent when no recycle is employed. Table I provided below sets forth an estimated overall material balance for such a process producing 660 million pounds per year of MTBE.

Table I

Estimated Overall Material Balance

Units Are in Lbs/Hr

|  | Hydrocarbon Feed | Methanol Feed | MTBE Product | Unreacted $C_4$'s |
|---|---|---|---|---|
| MTBE | -- | | 78,571 | |
| Isobutene | 51,435.1 | | -- | 1016.0 |
| Methanol | -- | 28,557.6 | -- | |
| Isobutane | 46,291.6 | | -- | 46,291.6 |
| Light Ends | 2,057.4 | | -- | 2,057.4 |
| Heavy Ends | 3,055.2 | | 3,055.2 | -- |
| Tert Butyl Alcohol | -- | | 288.4[2] | -- |
| Isobutene Dimer | -- | | 187.5 | -- |
| Water | 30.9 | 28.6 | -- | 19.8[1] |
|  | 102,870.2 | 28,586.2 | 82,102.1 | 49,384.8 |

(1) Hydrocarbon saturated with water in methanol extractor.

(2) Includes alcohol produced by reaction of water contained in recycle methanol.

The estimated MTBE analysis is as follows:

Table II

MTBE Product Analysis

($C_5$ Free Basis)

|  | Wt. % |
|---|---|
| MTBE | 99.4 |
| TBA | 0.37 |
| Methanol | < 5 ppm |
| Isobutane | < 5 ppm |
| Isobutene Dimer | 0.23 |
| Isobutene Trimer | < 5 ppm |
| Water | < 5 ppm |

The estimated unreacted $C_4$ analysis is as follows:

## Table III

### Unreacted $C_4$ Analysis
#### ($C_3$ Free Basis)

|  | Wt. % |
|---|---|
| Isobutene | 2.2 |
| Isobutane | 97.8 |
| Isobutene Dimer | < 5 ppm |
| TBA | < 5 ppm |
| MTBE | < 5 ppm |
| Methanol | < 5 ppm |
| Water | 420 ppm |
|  | 100 |

It should be noted that the above examples have been provided solely to illustrate some of the benefits and advantages of the present invention. Obviously, there are many variations and modifications that can be made without departing from the scope of the present invention.

Claims

1. A process for preparing methyl tertiary butyl ether (MTBE) wherein a fresh feedstream containing isobutene and at least one other hydrocarbon capable of forming an azeotrope with methanol is combined with methanol and subjected to an etherification reaction to produce an effluent containing MTBE and methanol, and the effluent is subjected to fractionation to produce a bottoms product comprising MTBE and an overhead product comprising methanol and unreacted hydrocarbons, c h a r a c t e r i z e d  b y  recycling to the etherification portions of the overhead that have not had the methanol separated therefrom.

2. A process according to claim 1 characterized in that the overhead from said fractionation is recycled to the etherification in an amount sufficient to insure that substantially all of the methanol in the etherification effluent will go overhead in the fractionation step.

3. A process according to claim 1 or 2 characterized in that said fresh feedstream consists essentially of $C_4$ hydrocarbons and the isobutene is present in said fresh feedstream in an amount greater than about 25 weight percent.

4. A process according to any of claims 1 to 3 characterized in that the etherification is carried out in two stages by feeding said fresh feedstream, methanol, and said recycled overhead through a first reaction zone comprising a fixed bed of acid ion exchange resin, said first reaction zone being operated under conditions such that there is at least about 80 percent conversion of said isobutene, and feeding effluent from said first reaction zone to a second reaction zone comprising a second fixed bed of acid ion exchange resin, said second reaction zone being operated under conditions such that there is at least 50 percent conversion of the remaining isobutene.

5. A process according to claim 4 characterized in that the molar ratio of methanol to isobutene in the total feed entering said first reaction zone is about 1/1.

6. A process according to claim 4 or 5 characterized in that said first and second reaction zones are operated under substantially adiabatic conditions.

7. A process according to any of claims 4 to 6 characterized in that effluent from said first reaction zone is recycled back through said first reaction zone.

8. A process according to any of claims 1 to 7 characterized in that said overhead from said fractionation is recycled in such an amount that the weight percent of azeotroping hydrocarbon in the feed resulting from the combination of the fresh feedstream and the overhead recycle is no less than about 60 weight percent on a methanol free basis.

19. A process according to any of claims 1 to 8 characterized in that said fresh feedstream comprises isobutylene and at least one hydrocarbon selected from the group consisting of isobutane, n-butane and linear butenes.

FIG. I

METHANOL

ISOBUTENE

C4 RAFFINATE

METHANOL

WATER

MTBE

WATER

0075838

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | --- | | C 07 C 43/04 |
| X | GB-A-2 043 065 (INSTITUT FRANCAIS DU PETROLE) * Whole document * | 1-6,8, 9 | C 07 C 41/06 C 07 C 41/42 |
| | --- | | |
| X | FR-A-2 272 065 (SNAM PROGETTI) * Examples 1,2 * & US - A - 4 071 567 | 1-6,8, 9 | |
| | --- | | |
| A | FR-A-2 450 799 (NIPPON OIL) * Page 16, line 8 - page 18, line 21 * & GB - A - 2 048 247 | 1,4,7 | |
| | ----- | | |
| | | | TECHNICAL FIELDS SEARCHED (Int. Cl. ³) |
| | | | C 07 C 41/00 C 07 C 43/00 |

The present search report has been drawn up for all claims

| Place of search THE HAGUE | Date of completion of the search 25-11-1982 | Examiner WRIGHT M.W. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82